# EUROPEAN PATENT APPLICATION

(11) **EP 4 721 645 A1**
(43) Date of publication of application: **08.04.2026**
(21) Application number: 24811324.3
(22) Date of filing: 13.05.2024
(51) Int. Cl.: A61B 5/00, A61B 5/026, G16H 50/20, G16H 50/50, G16H 30/20

(54) **ARTIFICIAL INTELLIGENCE MODEL-BASED AUTOMATED FLAP STATE EVALUATION METHOD AND ANALYSIS DEVICE**

(30) Priority: 24.05.2023 KR 20230066721
(71) Applicant: Samsung Life Public Welfare Foundation, Seoul 04348 (KR)
(72) Inventor: LEE, Kyeong Tae, Seoul 06351 (KR); KIM, Ze Ro, Seoul 06351 (KR); KIM, Da Eun, Seoul 06351 (KR); LEE, Sang Mee, Seoul 06351 (KR); CHUNG, Myung Jin, Seoul 06351 (KR)
(74) Representative: Ostertag & Partner Patentanwälte mbB
(86) International application number: PCT/KR2024/006480
(87) International publication number: WO 2024/242385

(57) **Abstract**

A flap condition assessment method using flap region images includes receiving, by an analysis device, a source image including a flap region of a subject, segmenting, by the analysis device, the flap region in the source image, and inputting, by the analysis device, the image of the segmented flap region into a classification model to output information indicating the condition of the flap region.

## Description

### [Technical Field]

The following description relates to a technique for evaluating the condition of a flap after flap surgery.

### [Background Art]

Flap surgery is a surgical procedure that transplants a flap removed from another site when skin tissue defects occur due to skin cancer resection surgery, trauma, or skin infection. In particular, free flap surgery includes surgery that connects blood vessels and nerves together with skin tissue. Therefore, monitoring the blood supply status of the flap transplanted through flap surgery is very important. If vascular compromise occurs, reoperation may be necessary. If it is not performed within the golden period, the success rate drops sharply.

### [Disclosure]

### [Technical Problem]

Currently, medical staff continuously monitor the condition or progress of the flap for a considerable period of time. Therefore, much time and manpower are consumed in managing the surgical site after flap surgery.

The technology described below seeks to provide an image-based monitoring system for flaps transplanted through flap surgery. The technology described below seeks to provide a system for evaluating the current condition of the flap. Furthermore, the technology described below seeks to provide a system for predicting the future condition of the flap.

### [Technical Solution]

In one general aspect, there is provided a method of flap condition assessment method using flap region images, the method including receiving, by an analysis device, a source image including a flap region of a subject; segmenting, by the analysis device, the flap region in the source image; and inputting, by the analysis device, the image of the segmented flap region into a classification model to output information indicating the condition of the flap region.

In another general aspect, there is provided an analysis device for evaluating flap condition, the analysis device including an interface device that receives a source image including a flap region of a subject; a storage device that stores a segmentation model for segmenting regions of interest and a classification model that receives flap region images and classifies flap conditions; and a computing device that segments the flap region in the source image using the segmentation model, and inputs the image of the segmented flap region into the classification model to output the condition of the flap region.

### [Advantageous Effects]

The technology described below may automatically monitor the condition of the flap transplanted through flap surgery based on images. Therefore, the technology described below may reduce medical personnel required to for monitoring flap conditions.

The technology described below may quickly provide information on the need for reoperation on the flap. That is, the technology described below contributes to increasing the success rate of flap surgery.

### [Description of Drawings]

FIG. 1 is an example of a flap condition monitoring system.
FIG. 2 is an example of a flap condition evaluation process.
FIG. 3 is an example of a process for constructing a dataset.
FIG. 4 is an example of a learning process of a learning model for flap condition evaluation.
FIG. 5 is an example of an analysis device for evaluating or predicting flap condition.

### [Modes of the Invention]

The technology described below may be modified in various ways and may have various embodiments, and specific embodiments will be illustrated in the drawings and described in detail. However, this is not intended to limit the technology described below to specific embodiments, and should be understood to include all changes, equivalents, and substitutes included in the spirit and technical scope of the technology described below.

Terms such as first, second, A, B, etc. may be used to describe various components, but the components are not limited by the terms, and are used only for the purpose of segmenting one component from another. For example, without departing from the scope of rights of the technology described below, a first component may be called a second component, and similarly, a second component may also be called a first component. The term and/or includes a combination of a plurality of related listed items or any one of the plurality of related listed items.

In the terms used in this specification, singular expressions should be understood to include plural expressions unless clearly interpreted differently in context, and terms such as "includes" mean that the described features, numbers, steps, operations, components, parts, or combinations thereof exist, and should be understood as not excluding the existence or possibility of addition of one or more other features, numbers, step operations, components, parts, or combinations thereof.

Before providing a detailed description of the drawings, it should be clarified that the division of components in this specification is merely divided according to the main function that each component is responsible for. That is, two or more components described below may be combined into one component, or one component may be divided into two or more according to more subdivided functions. In addition, each of the components described below may additionally perform some or all of the functions that other components are responsible for in addition to the main function that it is responsible for, and of course, some of the main functions that each component is responsible for may be exclusively performed by other components.

Also, in performing a method or operation method, each of the processes constituting the method may occur differently from the specified order unless the context clearly specifies a specific order. That is, each process may occur in the same order as specified, may be performed substantially simultaneously, or may be performed in the opposite order.

The technology described below is a technique for evaluating flap condition using images of a patient's flap area.

Hereinafter, source image means an image including a flap region where free flap surgery was performed. The flap region means the flap area that replaced the original skin tissue by performing free flap surgery. A source image means an image such as a photograph or a video frame.

Hereinafter, it will be explained that an analysis device evaluates or predicts flap condition using images including the flap region. The analysis device may be implemented as various devices capable of data processing. For example, the analysis device may be implemented as a PC, a server on a network, a smart device, a chipset with an embedded dedicated program, etc.

The analysis device may segment regions of interest in the source image using a learning model. The region of interest means the flap region. In addition, the analysis device may classify or evaluate flap condition using a learning model.

The training data construction and learning model construction process may be performed on a separate training device. The training device is a computer device capable of performing certain image processing and model learning process control. The training device may be implemented in the form of a PC, a server on a network, a smart device, a chipset with an embedded dedicated program, etc.

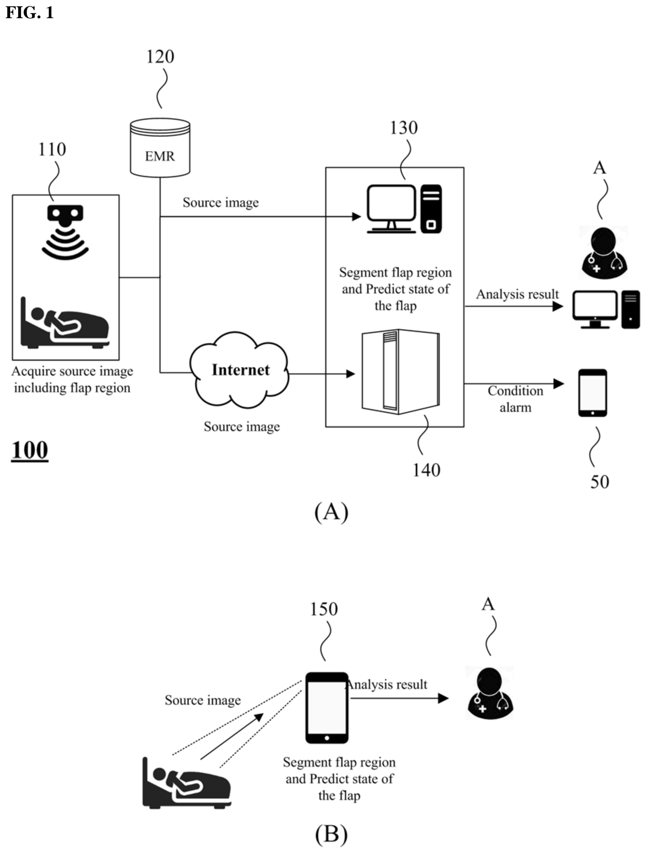

FIG. 1 is an example of a flap condition monitoring system (100).

FIG. 1(A) is an example of a system (100) composed of multiple devices. FIG. 1 shows an example where the analysis devices are a computer terminal (130) and a server (140).

A camera (110) or image sensor acquires a source image including the flap region of the subject (patient). The source image may generally include the flap region and the skin region surrounding the flap. In addition, the source image may further include patient clothing, medical equipment, gauze for protecting the affected area, background, etc.

The camera (110) may acquire source images continuously or at regular intervals.

The camera (110) may be a device built into a portable device such as a smartphone.

The camera (110) may store the acquired flap region images in an EMR (Electronic Medical Record, 120) or a separate database (DB) through wired or wireless networks.

User (A) (e.g., a clinician) may monitor the patient's flap condition. User (A) may analyze the source image using the computer terminal (130). The computer terminal (130) may receive the source image from the camera (110) or EMR (120) through wired or wireless networks.

In some cases, the computer terminal (130) may be a device physically connected to the camera (110). The computer terminal (130) may preprocess the source image consistently. The computer terminal (130) may segment the flap region, which is the region of interest, in the source image. The computer terminal (130) may analyze the segmented flap region using a learning model. The computer terminal (130) may classify the flap condition based on the value output by the learning model. In some cases, the computer terminal (130) may predict the future flap condition based on the value output by the learning model. User A may check the analysis results on the computer terminal (130).

The server (140) may receive flap region images from the camera (110) or EMR (120). The server (140) may preprocess the source image consistently. The server (140) may segment the flap region, which is the region of interest, in the source image. The server (140) may analyze the segmented flap region using a learning model. The server (140) may classify the flap condition based on the value output by the learning model. In some cases, the server (140) may predict the future flap condition based on the value output by the learning model. The server (140) may transmit the analysis results to user A's terminal. Alternatively, if the server (140) determines that the flap condition is abnormal (e.g., reoperation required), it may alert the flap condition to the medical staff terminal (50).

The computer terminal (130) and/or server (140) may also store the analysis results in the EMR (120).

FIG. 1(B) is an example of a flap condition monitoring system implemented in the form of a user terminal (150) such as a smartphone. User (A) may check the flap condition using the user terminal (150).

The user terminal (150) may capture a source image including the subject's flap region using a built-in camera. The user terminal (150) may analyze the source image using an installed application. The user terminal (150) may preprocess the source image consistently. The user terminal (150) may segment the flap region in the source image. The user terminal (150) may analyze the segmented flap region using a learning model. The user terminal (150) may classify the flap condition based on the value output by the learning model. In some cases, the user terminal (150) may predict the future flap condition based on the value output by the learning model. The user terminal (150) may output information about the flap condition.

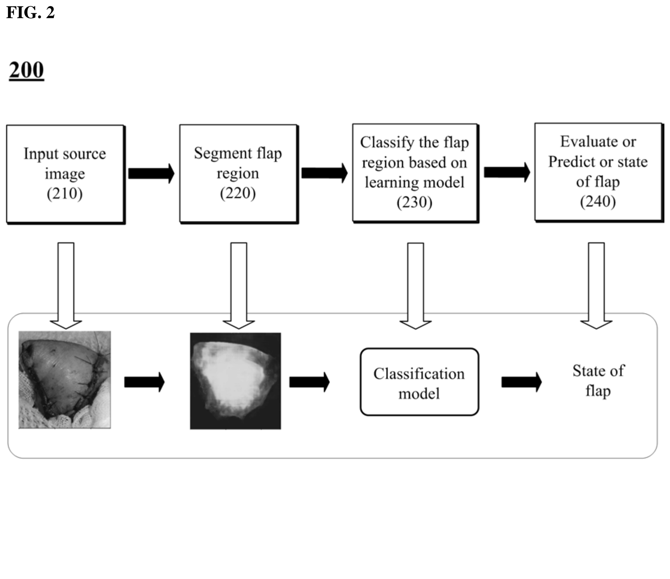

FIG. 2 is an example of a flap condition evaluation process (200).

The analysis device receives a source image including the flap region (210). The analysis device may preprocess the input flap region image consistently. Preprocessing may include noise removal, image size adjustment, image cropping, etc.

The analysis device may segment the flap region in the source image (220). The analysis device may segment the flap region in the source image using a certain image processing technique. Alternatively, the analysis device may segment the flap region in the source image using a trained segmentation model. For example, the segmentation model may be any one of a U-net based model, FCN (Fully Convolution Network), etc.

The analysis device analyzes the extracted flap region using a learning model (230). The model that analyzes the condition of the flap region may be called a classification model. The classification model may use any one of various types of models.

The classification model may be a regression analysis model that analyzes the trend of color values in the image. The color of the flap changes depending on the blood flow condition of the flap. If blood flow supply inside the flap is not smooth and thrombus accumulates in the blood vessels, the skin becomes red. Therefore, regression analysis may analyze the correlation between the area ratio of red color in the skin and the condition of the flap to classify the condition of the flap based on the currently input image.

Alternatively, the classification model may be a deep learning model that interprets images. For example, the classification model may be a CNN (Convolutional neural network)-based model. The trained CNN may extract features of the input flap region image and output probability values for the flap condition based on the extracted features. Of course, the classification model may be implemented as any one of various deep learning models such as vision transformers as well as CNN-based models.

The analysis device classification model may evaluate the current flap condition of the analysis subject based on the flap condition classification result or flap condition probability value (240). Alternatively, the analysis device classification model may predict future flap condition based on the flap condition classification result or flap condition probability value (240). Flap condition prediction is predicting the flap condition at a certain point after the time when the flap region image was taken. In the learning process, depending on the input data and ground truth (label values), the learning model may infer the current flap condition or future flap condition.

The process by which researchers built the model is explained. Researchers collected a dataset from patients who underwent free flap surgery at their affiliated medical institution from January 2020 to August 2023. The dataset includes retrospective data and prospective data. Retrospective data were collected from patients who underwent free flap surgery from March 2020 to February 2022. Retrospective data were collected by capturing 15 to 20 photographs per patient using smartphones. Retrospective data consisted of photographs with non-normalized image size, distance, and illuminance environment. Prospective data were collected for data normalization. Prospective data were additionally collected from patients who underwent free flap surgery from March 2022 to August 2023. Prospective data were collected using the same smartphone at a constant illuminance and fixed distance.

Table 1 below shows demographic information and flap information of the population used in dataset construction. Photographs including the flap region were collected from a total of 305 patients. The prospective data were collected from 203 patients, and the retrospective data were collected from 102 patients.

**[Table 1]**

| | | Total (n=305) | Retrospective (n=203) | Prospective (n=102) |
|---|---|---|---|---|
| **Patient demographics** | | | | |
| Age (median, range) | | 62 (8-86) | 62 (8-86) | 62 (13-95) |

| Sex | | | | |
|---|---|---|---|---|
| | Male | 178 (58.4%) | 124 (61.0%) | 54 (52.9%) |
| | Female | 128 (42.0%) | 79 (38.9%) | 49 (48.0%) |

| Race | | | | |
|---|---|---|---|---|
| | Asian | 302 (99.0%) | 201 (99.0%) | 101 (99.0%) |
| | Caucasian | 3 (1.0%) | 2 (1.0%) | 1 (1.0%) |

| Defect site | | | | |
|---|---|---|---|---|
| | Head and neck | 62 (20.3%) | 44 (21.7%) | 18 (17.6%) |
| | Trunk | 66 (21.6%) | 52 (25.6%) | 14 (13.7%) |
| | Extremity | 177 (58.0%) | 107 (52.7%) | 70 (68.6%) |

| Operation-related | | | | |
|---|---|---|---|---|
| Flap used | | | | |
| | ALT flap | 144 (47.2%) | 95 (40.8%) | 49 (48.0%) |
| | TDAP flap | 48 (15.7%) | 35 (15.0%) | 13 (12.7%) |
| | LD MC flap | 45 (14.8%) | 34 (14.6%) | 11 (10.8%) |
| | SCIP flap | 42 (13.8%) | 20 (8.6%) | 22 (21.6%) |
| | DIEP flap | 25 (8.2%) | 21 (9.0%) | 4 (3.9%) |
| | RASP flap | 8 (2.6%) | 8 (3.4%) | 0 |
| | Radial forearm free flap | 7 (2.3%) | 7 (3.0%) | 0 |
| | Fibular free flap | 3 (1.0%) | 3 (1.3%) | 0 |
| | DSAP flap | 3 (1.0%) | 3 (1.3%) | 0 |
| | Others | 10 (3.3%) | 7 (3.0%) | 3 (2.9%) |

The body parts where free flap surgery was performed were divided into Head and neck, trunk, and Extremity. Specific flap regions include ALT (anterolateral thigh), TDAP (thoracodorsal artery perforator), LDMC (latissimus dorsi myocutaneous), SCIP (superficial circumflex iliac artery perforator), DIEP (deep inferior epigastric perforator), and DSAP (dorsal scapular artery perforator).

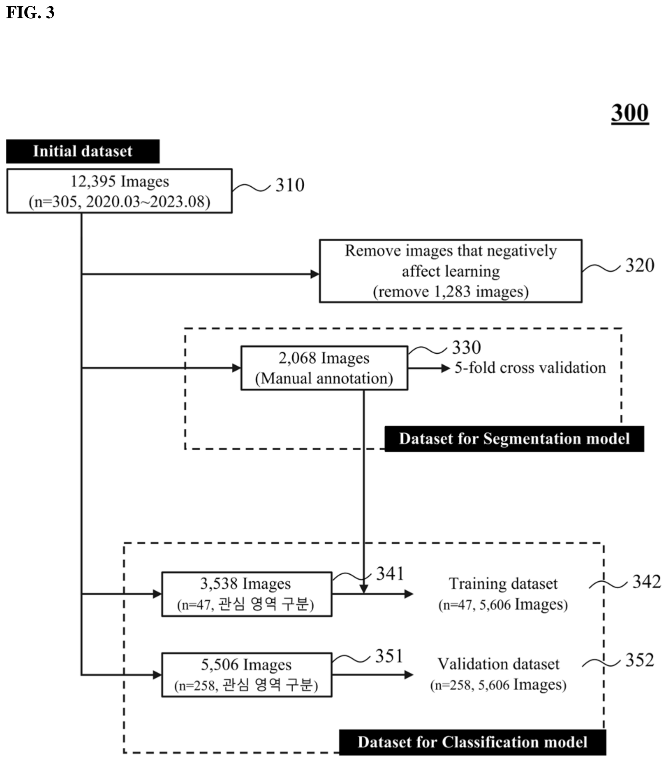

FIG. 3 is an example of a process (300) for constructing a dataset. It is explained that the training device performs the dataset construction process.

The training device receives an initial dataset (310). Researchers collected 12,395 images from the aforementioned 305 patients as an initial dataset.

The training device may remove images that may negatively affect learning from the initial dataset (320). The training device may remove images containing discoloration due to hematoma, etc. and images containing multiple flaps. Through this process, researchers removed 1,283 images to prepare a dataset of 11,112 images. The dataset of 11.112 consisted of 10,115 normal flaps (91%) and 997 flaps with abnormal perfusion status (9%).

The training device may construct a dataset for a segmentation model with images from the dataset where the flap boundary is clearly visible (330). Researchers extracted 2,068 images with clearly visible flap boundaries from the 11,112 images.

The dataset for the segmentation model includes original images and ground truth images in which the flap region, which is the region of interest, is manually segmented (manually annotated) for the corresponding original images.

The training device may construct training dataset and validation dataset for the classification model from the dataset.

The training device may select initial images for classification model training from the dataset. Researchers selected 3,538 images for 43 patients from the 11,112 images as training images. The training device may segment the region of interest (flap region) in the input image using the trained segmentation model. The training device may use the image with the segmented region of interest as a training dataset for the classification model (341). Furthermore, the training device may augment the training dataset. The training device may augment images through image flip, brightness adjustment (random delta adjustment), and contrast adjustment (342). In addition, the training device stores the ground truth (flap condition information) for images in the training dataset matched to the image (342). Therefore, the training dataset consists of flap region images and ground truth for the corresponding images. Researchers augmented 3,583 images to construct a training dataset of 5,606 images. Also, researchers determined ground truth by evaluating flap conditions through multiple medical experts.

The training device may select images for classification model validation from the dataset. Researchers selected 5,506 images for 258 patients from the 11,112 images as validation images. The training device may segment the region of interest (flap region) in the input image using the trained segmentation model. The training device may use the image with the segmented region of interest as a validation dataset for the classification model (351). Furthermore, the training device may augment the validation dataset. However, researchers used 5,506 images as is without augmenting the validation data. In addition, the training device stores the ground truth (flap condition information) for images in the validation dataset matched to the image (352). Therefore, the validation dataset consists of flap region images and ground truth for the corresponding images.

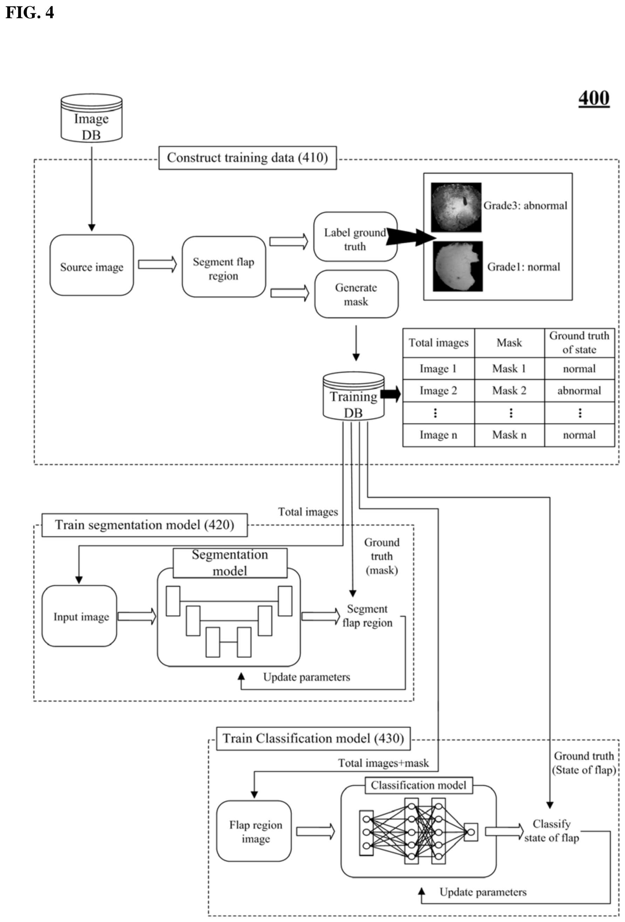

FIG. 4 is an example of a learning process (400) of a learning model for flap condition evaluation. The learning model construction process may be divided into a training data construction process (410), a segmentation model learning process (420), and a classification model learning process (430).

The training device constructs a training dataset (410). An image database (DB) stores source images including flap regions of the population. The training device may select source images to be used for learning from the image DB. The training device may segment the flap region in the source image. Flap region distinction may be performed manually. Alternatively, the training device may segment the flap region using a trained separate segmentation model. The training device may segment the flap region to generate a mask for the flap region. In addition, the training device may receive condition evaluation information for the flap region. Condition evaluation may be performed by medical experts as described above. For example, the condition of the flap may be divided into normal state (normal, grade 1) and abnormal state (abnormal, grade 3). The training device may store the final training dataset in the training DB. The training dataset may include the entire image, the flap region image or mask of the corresponding entire image, and condition information of the corresponding flap region.

The training device may perform a process of training the segmentation model (420). The segmentation model is a model that segments the flap region, which is the region of interest, in the source image. The segmentation model may be any one of various structured models. The training device constructs a segmentation model using the entire image (source image) and the flap region mask segmented from the corresponding image in the training DB. The training device inputs the entire image into the segmentation model. The training device compares the flap region segmented by the segmentation model with the ground truth to update the learning parameters of the model. Through this process, the segmentation model is trained to segment the flap region corresponding to the ground truth. The ground truth may be a flap region image or a flap region image with a mask applied to the entire image.

The training device may perform a process of training the classification model (430). The training device may construct a classification model using the segmented flap region image and the ground truth, which is the flap condition information. The classification model may be any one of various structured models.

The training device inputs the flap region image into the classification model. The training device compares the result output by the classification model (flap condition classification) with the ground truth to update the learning parameters of the model. Through this process, the classification model is trained to output condition information for the input flap region image.

The training device may repeatedly perform the learning process using the training data of the population.

The following explains the performance results of the model built by the researchers.

The validation results of the segmentation model are explained.

Researchers constructed and validated three CNN-based segmentation models. The three segmentation models used U-Net (refer to O. Ronneberger et al., U-net: Convolutional networks for biomedical image segmentation, in Medical Image Computing and Computer-Assisted Intervention-MICCAI, 2015), VGG16-UNet (refer to A.A. Pravitasari et al., UNet-VGG16 with transfer learning for MRI-based brain tumor segmentation, TELKOMNIKA Telecommunication, Computing, Electronics and Control, vol. 18, no. 3, pp. 1310-1318, 2020), and FS-Net (Flap Segmentation Network).

FS-Net is a CNN-based network, a model built by researchers for flap distinction. FS-Net has an encoder-decoder structure. The encoder receives an image of 256×256 pixel size and compresses it into a 16×16 size representation. The encoder includes successive 2D (dimension) convolutional layers with batch normalization and ReLU activation function. The decoder has a structure symmetrical to the encoder, but includes 2D transposed convolutional layers for upsampling. The decoder upsamples the 16×16 size representation to generate an image of 256×256 pixel size. The decoder normalizes pixel values using min-max normalization. FS-Net receives a source image including the flap region and outputs a mask corresponding to the flap region. The training device and/or analysis device may segment the flap region using the mask output by the segmentation model.

Researchers performed 5-fold cross-validation on the constructed segmentation model. Segmentation model performance was evaluated by comparing the results segmented by the segmentation model with the results manually segmented by experts (ground truth). Table 2 below shows the results of evaluating the performance of the segmentation model. Performance was evaluated using Jaccard Similarity and Dice Coefficient. The three segmentation models did not have a large performance difference, and FS-Net was relatively slightly higher in performance.

**[Table 2]**

| Models | Jaccard Index (mean ± SD) | Dice Coefficient (mean ± SD) | Specificity (mean ± SD) | Sensitivity (mean ± SD) |
|---|---|---|---|---|
| VGG16-U-Net | 0.913±0.007 | 0.955±0.004 | 0.987±0.002 | 0.956±0.004 |
| U-Net | 0.921±0.004 | 0.959±0.002 | 0.988±0.002 | 0.959±0.004 |
| FS-Net | 0.942±0.019 | 0.970±0.010 | 0.992±0.004 | 0.970±0.010 |

The performance validation results of the classification model are explained.

Researchers constructed and validated five CNN-based classification models. Four segmentation models used VGG16, ResNet50, InceptionV3, and DenseNet121. Furthermore, researchers additionally used one custom CNN model. The custom CNN model consists of 3 convolutional blocks, a dense layer, and a softmax layer. The convolutional block includes a 2D convolutional layer with ReLU activation function and max pooling layer. Researchers validated the performance of the classification model by comparing the inference results of the classification model with the ground truth. The performance of the classification models is shown in Table 3 below. All classification models showed high performance without significant differences.

**[Table 3]**

| Model | AUC (95% CI) | Sensitivity (95% CI) | Specificity (95% CI) | Specificity (of sensitivity 0.9) |
|---|---|---|---|---|
| VGG16 | 0.924 (0.908-0.939) | 0.78 (0.744-0.815) | 0.961 (0.955-0.966) | 0.677 |
| Custom CNN | 0.934 (0.923-0.946) | 0.9 (0.873-0.926) | 0.852 (0.842-0.863) | 0.852 |
| InceptionV3 | 0.94 (0.929-0.951) | 0.79 (0.753-0.826) | 0.956 (0.950-0.961) | 0.759 |
| ResNet50 | 0.943 (0.930-0.955) | 0.908 (0.884-0.933) | 0.857 (0.847-0.867) | 0.863 |
| DenseNet121 | 0.96 (0.950-0.969) | 0.91 (0.886-0.934) | 0.910 (0.903-0.918) | 0.909 |

In addition, researchers validated the performance of the classification model by classifying the site and type of flap where free flap surgery was performed, as shown in Table 4 below. Regardless of flap region and type, the classification model showed high performance.

**[Table 4]**

| Subgroup | | Rate of abnormal cases (%) | AUC (95% CI) | Sensitivity (95% CI) | Specificity (95% CI) |
|---|---|---|---|---|---|
| Flap type | | | | | |
| | ALT | 6.2% | 0.937 (0.911-0.961) | 0.839 (0.771-0.906) | 0.919 (0.907-0.931) |
| | LD MC & TDAP | 1.3% | 0.924 (0.826-0.980) | 0.909 (0.700-1.000) | 0.899 (0.878-0.920) |
| | SCIP | 12.0% | 0.963 (0.952-0.973) | 0.982 (0.967-0.996) | 0.794 (0.766-0.822) |
| | Others | 5.2% | 0.959 (0.940-0.977) | 1.000 (1.000-1.000) | 0.861 (0.831-0.892) |

| Defect site | | | | | |
|---|---|---|---|---|---|
| | Head & neck | 8.4% | 0.943 (0.923-0.963) | 0.849 (0.794-0.903) | 0.929 (0.912-0.945) |
| | Trunk | 2.5% | 0.997 (0.993-1.000) | 1.000 (1.000-1.000) | 0.966 (0.952-0.979) |
| | Extremity | 9.0% | 0.967 (0.959-0.974) | 0.980 (0.963-0.996) | 0.874 (0.860-0.887) |

The classification model whose performance was validated was a model that performs binary classification of the flap condition.

Furthermore, the classification model may be constructed as a model that performs multi-classification of the flap condition. Multi-classification may be three or more grades or scores for flap condition.

In addition, the classification model may be constructed as a model that predicts the future condition of the flap rather than the current condition. In this case, the training data may include the flap region at the current time point and the value of ground truth for the future time point condition of the corresponding flap region.

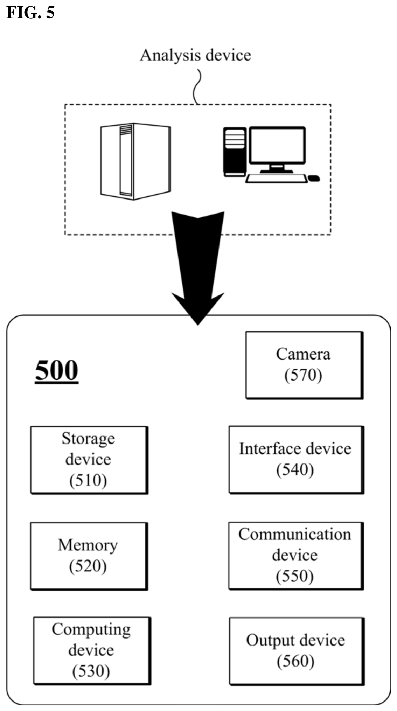

FIG. 5 is an example of an analysis device (500) for evaluating or predicting flap condition. The analysis device (500) corresponds to the aforementioned analysis device (130, 140, or 150 in FIG. 1). The analysis device (500) may be physically implemented in various forms. For example, the analysis device (500) may have the form of a computer device such as a PC, a server on a network, a chipset dedicated to data processing, etc.

The analysis device (500) may include a storage device (510), memory (520), computing device (530), interface device (540), communication device (550), and output device (560).

The storage device (510) may store source images including flap regions captured by the camera.

The storage device (510) may store a segmentation model that segments the flap region in the source image. The segmentation model is a model trained through the aforementioned process.

The storage device (510) may store image processing algorithms or programs that segment the flap region in the source image.

The storage device (510) may store flap region images obtained by segmenting the flap region in the source image.

The storage device (510) may store code or programs that analyze flap region images to classify flap condition.

The storage device (510) may store a classification model that receives the segmented flap region image and analyzes flap condition. The classification model is a model trained through the aforementioned process.

The storage device (510) may store the results of classifying or predicting the condition of the flap included in the input source image.

The memory (520) may store data and information generated during the process in which the analysis device analyzes the flap region image.

The interface device (540) is a device that receives certain commands and data from outside.

The interface device (540) may receive source images including flap regions from physically connected input devices or external storage devices.

The interface device (540) may also transmit the results of flap condition classification to external objects.

The interface device (540) may be a device that delivers data or information received through the communication device (550) inside the analysis device.

The communication device (550) means a component that receives and transmits certain information through wired or wireless networks.

The communication device (550) may receive source images including flap regions from external objects.

The communication device (550) may also transmit the results of flap condition classification to external objects such as user terminals.

The output device (560) is a device that outputs certain information.

The output device (560) may output interfaces necessary for data processing processes, flap regions, flap condition analysis results, etc.

Meanwhile, the analysis device (500) may further include a camera (570). The camera (570) may capture the flap region of a patient who underwent free flap surgery. In this case, the interface device (540) may deliver the source image captured by the camera to the storage device (510).

The computing device (530) may segment the flap region in the source image using image processing algorithms or programs.

Alternatively, the computing device (530) may segment the flap region by inputting the source image into the segmentation model. The computing device (530) may segment the flap region in the source image using the mask output by the segmentation model.

The computing device (530) may classify the flap condition by analyzing the segmented flap region image. The computing device (530) may classify the flap condition by inputting the segmented flap region image into the classification model.

Alternatively, the computing device (530) may predict the prognosis of the flap by inputting the segmented flap region image into the classification model. In this case, the classification model is a model trained to predict the flap condition at a future time point.

The computing device (530) may be a device such as a processor, AP, or chip with an embedded program that processes data and performs certain operations.

In addition, the flap image processing method, learning model construction method, and flap condition classification method as described above may be implemented as a program (or application) including an executable algorithm that can be executed on a computer. The program may be stored and provided in a transitory or non-transitory computer readable medium.

Non-transitory readable medium means a medium that stores data semi-permanently and can be read by a device, rather than a medium that stores data for a short moment such as registers, cache, memory, etc. Specifically, the various applications or programs described above may be stored and provided in non-transitory readable media such as CD, DVD, hard disk, Blu-ray disk, USB, memory card, ROM (read-only memory), PROM (programmable read only memory), EPROM (Erasable PROM, EPROM) or EEPROM (Electrically EPROM) or flash memory.

Transitory readable media means various RAMs such as Static RAM (SRAM), Dynamic RAM (DRAM), Synchronous DRAM (SDRAM), Double Data Rate SDRAM (DDR SDRAM), Enhanced SDRAM (ESDRAM), Synclink DRAM (SLDRAM), and Direct Rambus RAM (DRRAM).

The present embodiment and the drawings attached to this specification only clearly show part of the technical ideas included in the aforementioned technology, and all modified examples and specific embodiments that can be easily inferred by those skilled in the art within the scope of the technical ideas included in the specification and drawings of the aforementioned technology are obviously included in the scope of rights of the aforementioned technology.

## Claims

1. A flap condition assessment method using flap region images, the method comprising:
receiving, by an analysis device, a source image including a flap region of a subject;
segmenting, by the analysis device, the flap region in the source image; and
inputting, by the analysis device, the image of the segmented flap region into a classification model to output the condition of the flap region.

2. The method of claim 1, wherein the analysis device segments the flap region in the source image using a segmentation model.

3. The method of claim 1, wherein the classification model evaluates the condition of the flap region based on the red area ratio of the flap region.

4. The method of claim 1, wherein the classification model is a deep learning model that receives the image of the flap region and classifies the condition of the flap region.

5. The method of claim 1, wherein the classification model is a deep learning model that receives the image of the flap region and predicts the condition of the flap region at a future time point.

6. An analysis device for evaluating flap condition, the device comprising:
an interface device configured to receive a source image including a flap region of a subject;
a storage device configured to store a segmentation model for segmenting regions of interest and a classification model that receives flap region images and classifies flap conditions; and
a computing device configured to segment the flap region in the source image using the segmentation model, and input the image of the segmented flap region into the classification model to output the condition of the flap region.

7. The device of claim 6, wherein the classification model evaluates the condition of the flap region based on the red area ratio of the flap region.

8. The device of claim 6, wherein the classification model is a deep learning model that receives the image of the flap region and classifies the condition of the flap region.

9. The device of claim 6, wherein the classification model is a deep learning model that receives the image of the flap region and predicts the condition of the flap region at a future time point.
